# EUROPEAN PATENT SPECIFICATION

(11) **EP 2 403 410 B1**
(45) Date of publication and mention of the grant of the patent: **31.10.2018**
(21) Application number: 10749314.0
(22) Date of filing: 04.03.2010
(51) Int. Cl.: A61B 6/04, A61B 90/11, A61B 90/17

(54) **NEEDLE BIOPSY COMPRESSION PADDLE SYSTEM**
PADDELSYSTEM FÜR NADELBIOPSIEKOMPRESSION
SYSTÈME DE PLAQUE DE COMPRESSION POUR BIOPSIE À L'AIGUILLE

(30) Priority: 06.03.2009 US 158180 P
(43) Date of publication of application: 11.01.2012
(73) Proprietor: Hologic Inc., Marlborough, MA 01752 (US)
(72) Inventor: DEFREITAS, Kenneth, Patterson, NJ 12563 (US); LAVIOLA, John, Orange, Connecticut 06477 (US); VERMEULEN, Henri, B-1330 Rixensart (BE); URBAIN, Marc, 1325 Caheumont-Gistoux (BE); NICOSON, Zachary, Indianapolis, Indiana 46220 (US); PICKETT, Kathleen, Uncasville, Connecticut 06382 (US); GKANATSIOS, Nikolaos, Danbury, Connecticut 06810 (US)
(74) Representative: Hoyng Rokh Monegier LLP
(86) International application number: PCT/US2010/026167
(87) International publication number: WO 2010/102087

(56) References cited:
- EP-A1- 1 844 712
- EP-A1- 1 852 070
- FR-A1- 2 666 217
- US-A- 4 798 212
- US-A- 5 056 523
- US-A- 5 594 769
- US-A- 5 855 554
- US-A- 5 983 123
- US-B2- 7 496 398

## Description

### Related Applications

This application claims priority under 35 U.S.C. $1.119(e) to U.S. Provisional Application serial number 61/158,180, filed March 6, 2009.

### Background

Stereotactic biopsy is a biopsy procedure that uses a computer and imaging performed in at least two planes to localize a target lesion (such as a tumor or micro-calcifications in the breast) in three-dimensional space and guide the removal of tissue for examination by a pathologist under a microscope. Stereotactic core biopsy makes use of the underlying principle of parallax to determine the depth or "Z-dimension" of the target lesion. Stereotactic biopsies can be performed with the patient in a prone position on a prone biopsy table or with the patient in an upright, seated position. A known device for performing stereotactic biopsy is described in US Patent US 5,056,523. Said device comprises a compression paddle including a compression platform with passages for allowing a probe to pass through the passages and penetrate the breast. Said device further comprises complex localization equipment such as a probe guide support system to enable alignment of the probe with respect to a target region on the breast of a patient.

Upright stereotactic biopsy capability is typically added to an existing imaging system by mounting a stereotactic needle assembly onto an existing x- ray mammography machine. The stereotactic needle assembly may include a separate detector, compression capability and other high precision instrumentation. The expense of the needle biopsy assembly and the expertise required for its use limits its use to sophisticated operating environments.

### Summary

The present invention provides for an assembly of a compression paddle and a detachable needle guide having one or more guide holes extending therethrough, the holes being arranged for insertion of one or more biopsy devices, wherein the compression paddle comprises: a biopsy window; a support structure positioned across the biopsy window, wherein the support structure comprises a grid that is sized to accept the detachable needle guide, and wherein the grid is further adapted to adjustably position the needle guide within the biopsy window; a connection mechanism for attaching the compression paddle to a compression mechanism of an upright x-ray imaging system such that a plane of the compression paddle is horizontal; and wherein the compression paddle and needle guide include mechanical means for enabling the needle guide to be latched onto and/or introduced on the compression paddle such that its through holes extend parallel to the plane of the compression paddle, allowing for biopsying a breast from the side when the breast is compressed by the compression paddle and attached to the upright x-ray imaging system. Such an assembly of a compression paddle and needle guide may be used for performing breast needle biopsies. The assembly of compression paddle and needle guide uses low cost, disposable components and leverages the high performance imaging capabilities of present mammography or tomosynthesis system to achieve results similar to those provided by dedicated upright stereotactic assemblies having dedicated, high precision instrumentation. The assembly of compression paddle and needle guide may be packaged in a disposable kit including, an introducer sheath, an introducer stylet and an obturator. Such a disposable kit may be distributed with a biopsy needle of varying types including a core biopsy needle and vacuum assisted biopsy needle. In some embodiments, the compression paddle may include further detachable components, including a detachable grid or other feature which may be used to stabilize the needle, or simply the breast, during biopsy. With the assembly of compression paddle and detachable needle guide, the existing capability of an imaging system to provide high quality volume data is leveraged with the use of low cost needle positioning or breast stabilizing components to make needle biopsy capability available to a wide range of patient environments.

An assembly according to the invention is defined in appended independent claim 1. An assembly according to the preamble of claim 1 is known from US 5855554 A1.

### Brief description of the Figures

Figure 1 illustrates an x-ray imaging system with which the needle biopsy kit may be used;
Figures 2-3 illustrate compression paddles and detectors that may be provided in a breast imaging system;
Figures 4-9 illustrate various embodiments of compression paddles including features for supporting a needle guide during a breast biopsy;
Figures 10A-10C illustrate one embodiment of a lateral attachment mechanism that may be used to coupled a lateral needle guide to a compression paddle for performing a lateral biopsy;
Figure 11 illustrates an alternative embodiment of a compression paddle including a multi-dimensional sliding support for positioning the needle guide;
Figures 12A and 12B illustrate a compression paddle having an opening for accepting a disposable, detachable grid for stabilizing the breast during a biopsy; and
Figures 13 and 14 illustrate different views of an alternative embodiment of a detachable grid for use in the present invention.
Figure 15 illustrates various components of a needle biopsy introducer kit which may be provided with any of the compression paddles of the invention; and
Figure 16 is a flow diagram illustrating exemplary steps that may be performed during a breast biopsy using the compression paddle and other disposable components of the present invention.

### Detailed Description

According to one aspect of the invention a needle biopsy system includes a compression paddle having means for supporting a biopsy needle guide. The compression paddle is adapted for releasable coupling to a breast imaging system capable of obtaining either two-dimensional x-ray images or a three-dimensional x-ray image of a patient's breast. In an exemplary embodiment the compression paddle is molded from plastic and includes means for positioning the biopsy needle guide at a desired location. The compression paddle may be provided as part of a needle biopsy kit that includes one or more of a needle guide, a localizing obturator, an introducer sheath, an introducer stylet and a marker assembly. The biopsy kit components are advantageously fabricated from a low cost, radiolucent material which may be disposed subsequent to use. In one embodiment, the present invention provides an additional mode of use of biopsy kit components that are provided for Magnetic Resonance Imaging (MRI) assisted biopsies. As will be described in more detail below, the improved compression paddle design allows needle biopsies to be performed accurately and with increased speed using existing breast imaging equipment and a low cost, disposable kit, thereby extending the opportunity for needle biopsies into cost-constrained patient venues.

The advances that have recently been achieved in breast imaging are an enabling aspect of the present invention. Improvements in breast imaging software and hardware enable accurate Z-axis information to be obtained using stereotactic information in combination with a zero axis scout image using a standard 2D mammography device.

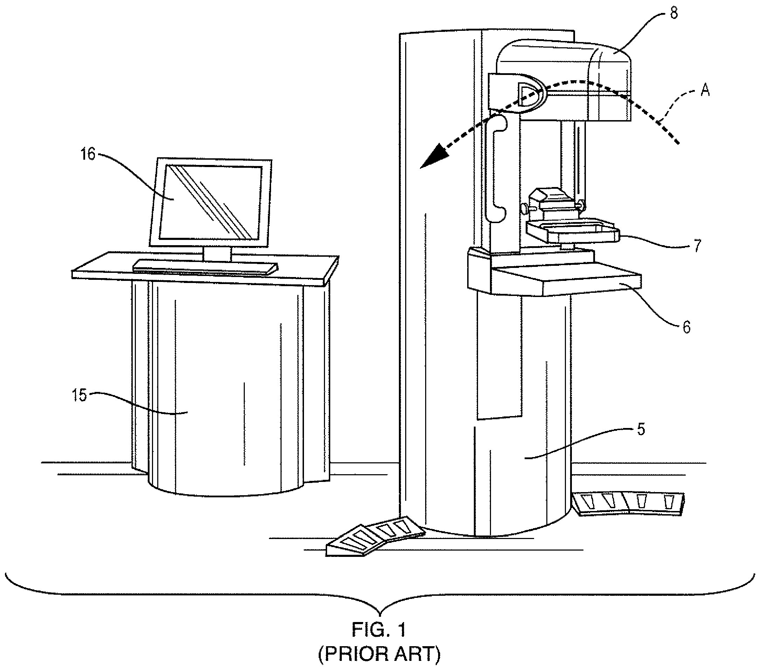

Precise X, Y and Z-axis information can also be obtained by obtaining a three-dimensional image of the breast using breast tomosynthesis imaging devices. Breast tomosynthesis is a three-dimensional imaging technology that involves acquiring x-ray images of a stationary compressed breast at multiple angles during a short scan. For example, referring to Figure 1, a tomosynthesis scan may follow the arc indicated by the dashed path A, taking a number of x- ray projection images along the way. The individual images are then reconstructed into a series of thin, high-resolution slices that can be displayed individually or in a dynamic cine mode.

Reconstructed tomosynthesis slices reduce or eliminate the problems caused by tissue overlap and structure noise in single slice two-dimensional mammography imaging. Digital breast tomosynthesis also offers the possibility of reduced breast compression, improved diagnostic and screening accuracy, fewer recalls, and 3D lesion localization. Examples of breast tomosynthesis systems are described in U.S. Patent Applications 7,245,694 and 7, 1 23,684, commonly owned by the Assignee of this application.

The compression paddle and disposable components described herein may be used with any 2D, 3D or combination breast imaging device for the purpose of needle biopsies. Traditionally x-ray needle biopsies have been performed using expensive, high precision stereotactic equipment. Magnetic Resonance
Imaging (MRI) compatible devices have been developed that allow vacuum- assisted needle biopsy to be performed under MR guidance. During the biopsy, a patient is positioned on a breast coil which raises the patient and allows the breast to be pendulant. A series of MR images are obtained to locate the lesion, insert and image the localization obturator prior to biopsy, and image to verify removal of the lesion. The time needed to perform the MFU imaging is extensive, and the MRI equipment is expensive.

in contrast to MRI imaging, x-ray imaging may be performed relatively quickly. The compression paddle of the present invention allows needle biopsies to be performed at a fraction of the time and expense associated with MRI or stereotactic biopsies of the art.

Figure 1 illustrates a combination mammography/tomosynthesis imaging system which includes a control workstation 15 coupled to an imaging device 5. In one embodiment, the imaging device is a multi-mode imaging device capable of acquiring both two dimensional breast image data and three dimensional breast image data as described in U.S. Patent Application serial number 11/791,601, filed 11/23/2005. The workstation 15 includes a display 16 and a user interface which allows a radiologist to control the acquisition of and display of x-ray images using the imaging device 5. The imaging device includes an x-ray tube head 8, a compression assembly and compression paddle 7 and a receptor housing 6.
During acquisition of 2D or 3D images, a patient's breast is compressed between the compression paddle 7 and the receptor housing. Gamma rays emitted from the x-ray source are directed through the compression paddle and breast, and received at a digital detector (not shown) within the receptor housing 6. Data from the digital detector is read out and processed by software at the workstation 15 to generate viewable x-ray images. The images acquired by the scan may be viewed on the display 16. Depending upon the imaging mode used (mammography or tomosynthesis), the images viewed may include 2D images and/or 3D reconstructed slices. The 3D reconstructed image data can be obtained and processed into tomosynthesis images for display and/or storage as described, for example in co-pending patent application Ser. No. 10/723,486 or in U. S Provisional Application No. 60/628,516, filed Nov. 15, 2004.

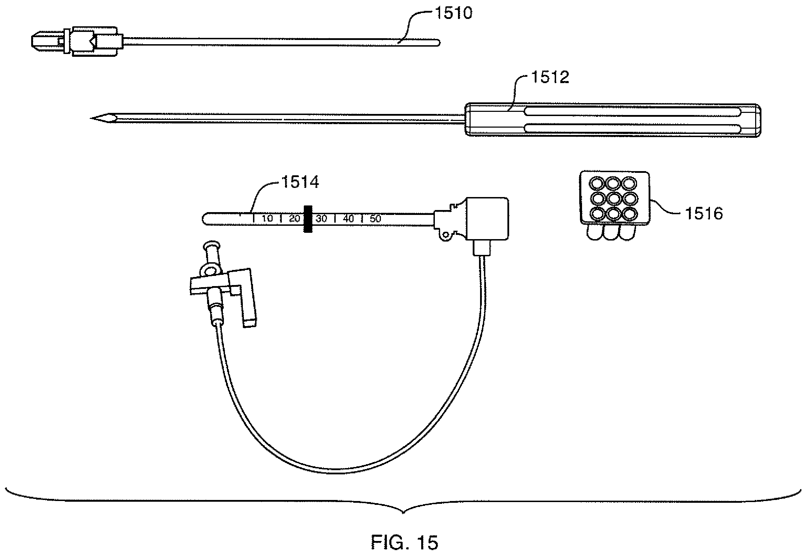

In one embodiment of the present invention, a user interface of the workstation is adapted to support a needle biopsy workflow such as that outlined in Figure 15. In particular, the user interface provides options for acquiring a biopsy scan to identify lesion, needle and marker location.

Before initiating the needle biopsy procedure, the imaging compression paddle 7 is replaced by a biopsy compression paddle of the present invention. Several embodiments of a biopsy compression paddle are described herein. One feature that is common to the various described embodiments is that the
biopsy compression paddles include a means for supporting a needle guide which includes one or more holes for insertion of one or more biopsy devices.

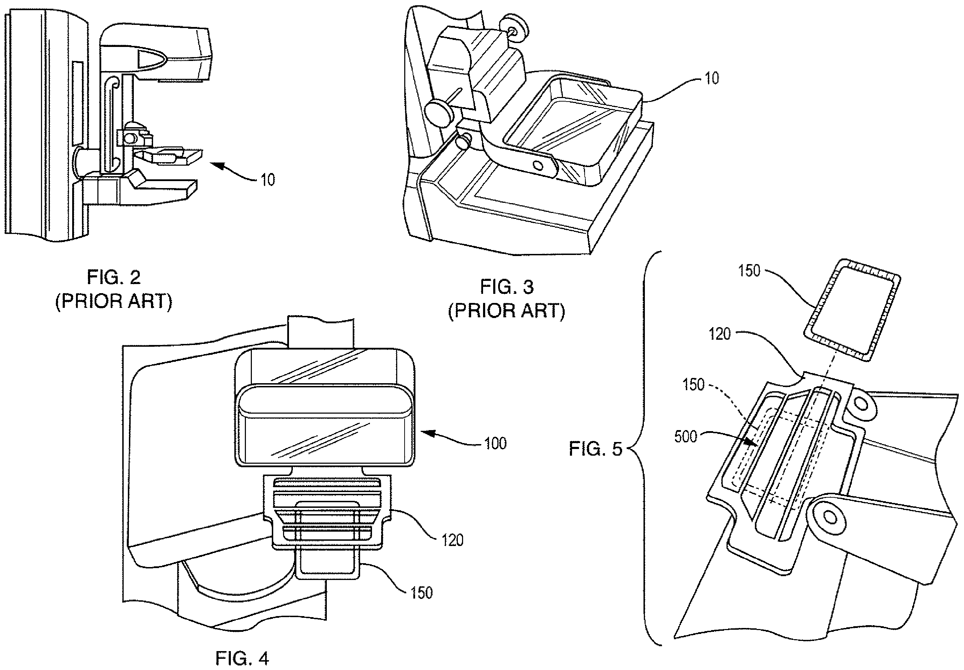

Figure 2 and 3 illustrate an embodiment of a typical compression paddle that is used for breast imaging. One embodiment of a compression paddle of the present invention is illustrated in Figures 4 and 5. The paddle 100 includes a support structure 120 comprised of needle guide frame comprising a plurality of spaced lateral ribs. The spacing of the lateral ribs is selected in accordance with dimensions of a needle guide block 500. That is the spacing is selected so that a needle guide 500 may be extended through the spaced lateral ribs and tightly supported by the ribs. The compression paddle 100 also includes an x/y positioning frame 150. In the embodiment of Figures 4 and 5 the needle guide frame is shown in exploded detail to include grid lines which assist in positioning the needle guide at a desired x/y location within a biopsy field. In one embodiment, the grid lines of the positioning frame are at least marginally radioopaque, allowing them to be viewed on the resultant x-ray image. The needle guide frame 120 is preferably made from a radiolucent material such as plastic or poly silicon or the like. In the embodiment illustrated in Figures 4 and 5, the ribs are parallel to a plane defined by the scanning motion of the x-ray tube head during tomosynthesis imaging in order to reduce blurring in the resulting three-dimensional image.

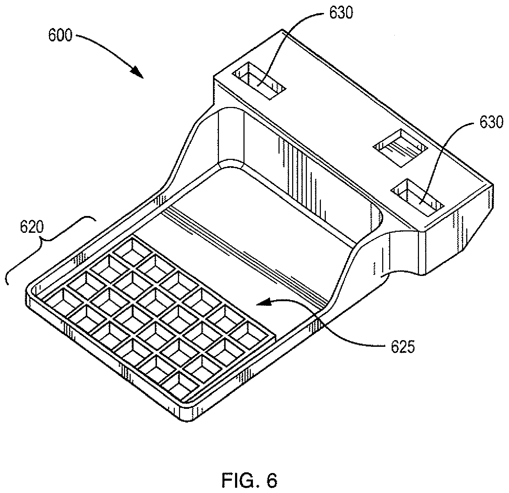

Figure 6 illustrates an alternate embodiment 600 of a compression paddle of the present invention. The compression paddle 600 is of the general shape and form of an imaging compression paddle 10 (Figures 2 and 3) and includes slot 630 which engage with latches of the compression system of the imaging device to secure the paddle onto the imaging device. Although slots are shown, it is appreciated that many different systems use different connection mechanisms for coupling the compression paddle to the compression system of the imaging device and the connector is not a limitation of the present invention.

The compression paddle of the present invention differs from conventional compression paddles generally used for upright biopsy for at least the reason that it includes a support structure 620, referred to herein as a biopsy needle support grid. Like the support structure of paddle 120 of Figures 4 and 5, the paddle 600 includes means for supporting and positioning the needle guide, where the dimensions of each grid opening 625 are matched to the size of the needle guide. The grid allows the needle guide to be placed in a selected x, y location of the breast. Such an arrangement increases the accuracy and reliability of needle guide placement by removing the opportunity for shifting of the needle guide during the biopsy procedure.

And additional advantage of the grid arrangement of the paddle 600 is that it increases the rigidity of the compression paddle, enabling the necessary breast compression to be realized for x-ray imaging. In prior art stereotactic imaging systems, a compression paddle is provided with a single enlarged opening that is positioned over the area to be biopsied. Because of the compression that is applied to the breast, the breast tissue "pooches" or expands upward, through the opening. As a result, adjustments to the reference point that is used for imaging and biopsy must be made. In contrast, the grid openings of the present invention are sufficiently small that pooching is minimized and no adjustments to reference planes need be made.

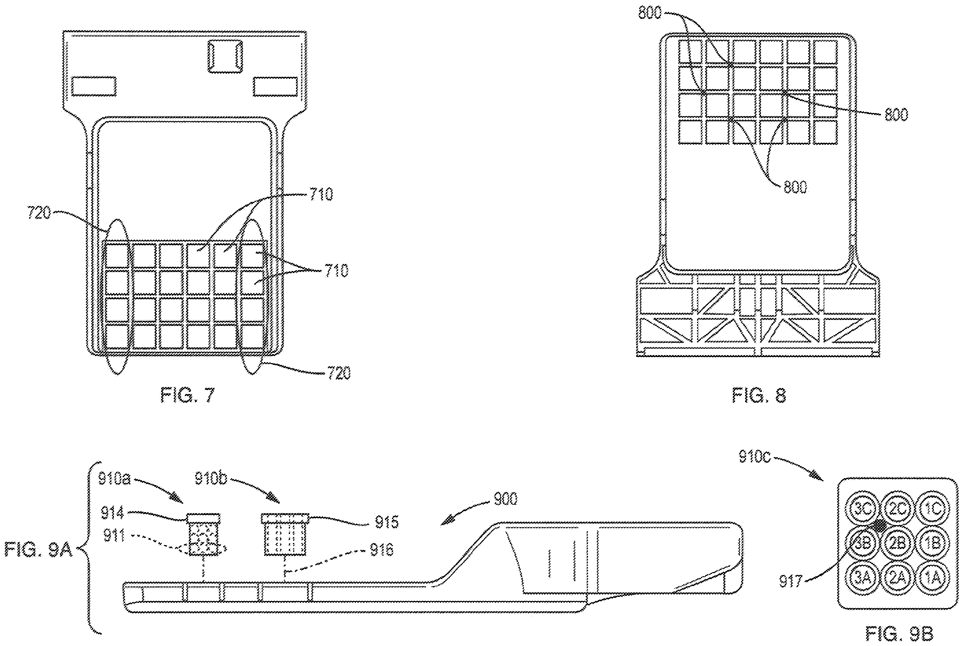

Figures 7, 8 and 9 illustrate the compression paddle 600 at varying perspectives. One feature of the compression paddle 600 is that is allows a needle guide 910 to be positioned in the support grid portion with at least two orientations, indicated as 910a and 910b in Figure 9. In general, a needle guide is a block of radiolucent material (such as plastic or polysilicon) having a number of holes extending therethrough. The holes are sized to accommodate passage of a biopsy needle and additional biopsy equipment. The needle guide is in general provided as a disposable component in a needle biopsy kit.

The needle guide in some embodiments includes a lip 914, 915. The exterior dimensions of the lip are sized larger than the grid holes 625 so that when the needle guide is inserted into the grid hole it will be precluded from extending falling through the grid. One method of positioning the needle guide is to extend the bottom of the needle guide into the grid hole until the lip portion engages a top surface of the grid, thereby fixedly supporting the needle guide.

A needle guide that may be used to biopsy breast tissue from a position on top of the breast is needle guide 910b. Needle guide 910b is similar to needle guides provided in an MRI kit. In the figures an alternate embodiment of the exemplary needle guide is shown to include a multitude of guide holes , and any number of holes, from one and up, can be selected as a matter of design preference. Once a location of the lesion and hence the appropriate grid hole is identified, the needle guide 910b is inserted into the grid as shown by arrow 916.

An alternate needle guide that may be used for laterally biopsy of the breast is needle guide 910a. Needle guide 910a is molded so that when the needle guide is inserted into one of the grid holes, the holes of the needle guide extend generally parallel to the plane of the compression paddle, rather than perpendicularly as described above. The needle guide 910a is pushed through the grid, such that at least one row (911) of guide holes extends below the compression plate. The needle guide 910a is thus preferably used in either of the rows 720 of the compression plate grid 620.

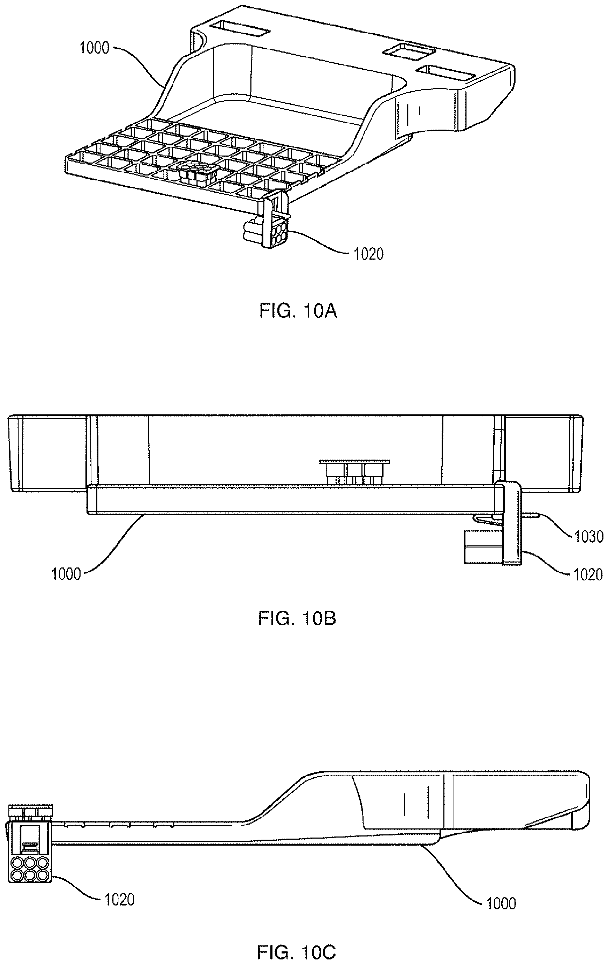

In an alternate embodiment of the present invention, the compression plate may be molded with slots on the edges, such as slots 900. A needle guide may include latches or other mechanical means for sliding down the slot until at
least a row of the needle guide is provided below the compression plate. In still an alternate embodiment, the compression plate and needle guide may include mechanical means for enabling the needle guide to be latched onto a desired location on the compression plate, such that one or multiple rows of through holes is made available for biopsying the breast from the side. For example, referring now briefly to Figures 10A-10C, a lateral needle guide 1020 includes a latch 1030 for coupling the lateral needle guide to the edge of the compression paddle 1000. Figure 10A is a perspective view of the paddle having the lateral needle guide latched into position. Figure 10B is a straight on view of the compression paddle with latched lateral needle guide, illustrating how the needle guide extends below the paddle. Figure 10C is a side view of the compression paddle with attached lateral needle guide. Alternative methods for coupling a lateral needle guide to the compression paddle, including slots, tabs, snaps and the like are considered equivalents hereto.

Referring back to Figure 8, in one embodiment the grid area of the compression plate may include one or more embedded fiducials 800. A fiducial is a radioopaque bead made, for example, from lead or other material. It is known that during compression, the reactive force of the breast pushes against the compression paddle so that the paddle is not always parallel to the surface of the receptor housing, but rather follows the general shape of the breast tissue and may therefore be tilted at an angle. It is important to understand the angle of tilt of the compression paddle as it may angularly offset the path of insertion of the biopsy needle. Information regarding the angular of tilt of the compression paddle may be determined by imaging the fiducials on the compression paddle. The information can be used during lesion localization and targeting to thereby increasing the accuracy of the biopsy procedure.

According to another aspect of the invention one or more fiducials may also be embedded in the needle guide, advantageously on the underside of the needle guide. Element 910c is a picture of a bottom end of a needle guide, and is shown to include fiducial 917. The advantage of providing a fiducial on the bottom of the needle guide block is that it adds further information to the radiologist as the radiologist scans through the three-dimensional representation of the breast.

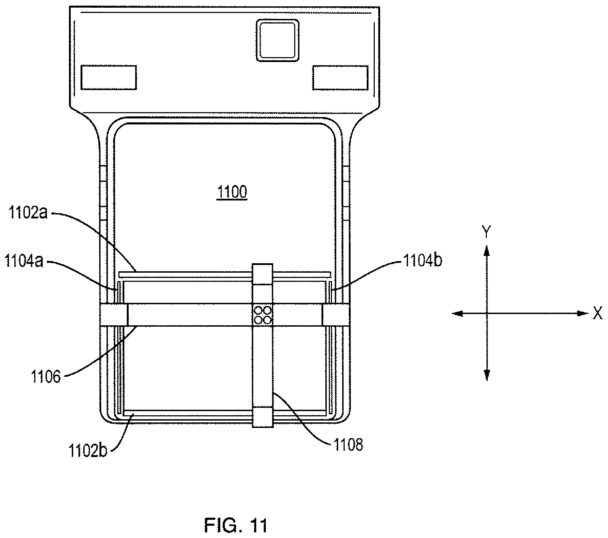

An alternate 'compass style' support structure is shown in Figure 11. The compression paddle includes longitudinal tracks 1104a and 1104b, upon which rails 1106 can traverse along the Y axis. The compression paddle also includes tracks 1102a and 1102b along which rail 1108 can traverse along the X axis. The tracks 1104, 1102 and rails 1108 and 1106 are preferably radiolucent. The intersection of rails 1108 and 1106 defines a space through which the needle guide may be inserted. The needle guide, positioned at the intersection of rails 1106 and 1108, can be positioned by appropriate movement of the guide within the rails. The rails advantageously can be locked at various positions along the track to secure the rails, and therefore the needle guide, when the guide is positioned over the lesion. The rails are advantageously made of a stiff plastic or polycarbonate material to provide rigid support for the needle guide. In contrast to the paddles of Figures 5 and 6, the rails present less visual interference in the resulting image. The rails may be advantageously detachable from the compression paddle, although it is appreciated that detachability of the compass mechanism is not a requirement.

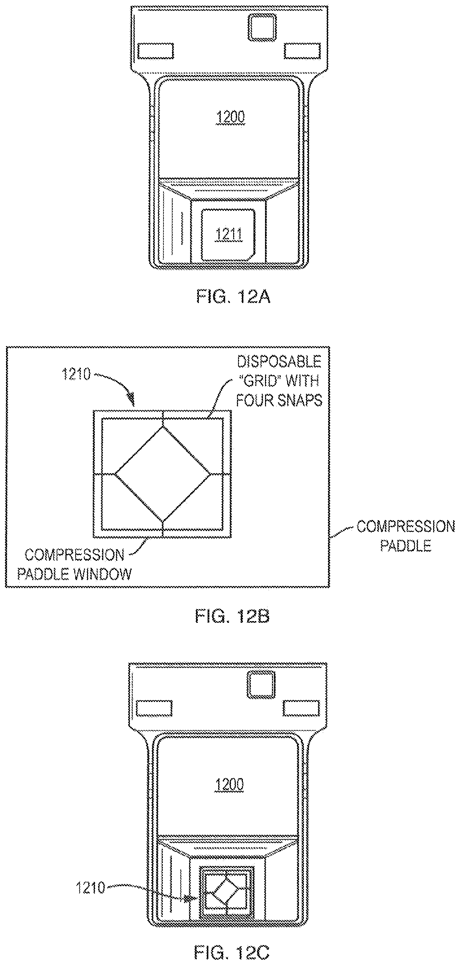

Although the compression paddle embodiments above have specifically discussed the use of the needle guide for performing biopsies using x-ray imaging modes, it is understood that there are a variety of imaging modes that may be used during breast biopsy, that there are a variety of biopsy needle types (fine needles, core needles and vacuum assisted core needles) and that a needle guide may not always be used. While the features of the compression paddle are useful for supporting the needle guide, that is not their sole utility. As discussed above prior art compression paddles that have been used for breast biopsy generally have an opening through which the portion of the breast to be biopsy is exposed. An example of one such paddle 1200 is illustrated in Figure 12A, with the opening indicated generally as feature 1211. The pressure of the compression paddle on the breast during biopsy causes the exposed area of the breast to 'pooch' out. The lack of compression within the biopsy window may permit movement of the lesion during the biopsy procedure. The grids of the compression paddles described above, while serving the purpose of supporting the needle guide, additionally serve the purpose of increasing the stability of the tissue to be biopsied and minimizing movement of the lesion.

In alternate embodiments of the invention the compression paddle opening therefore need not be sized to accommodate a needle guide, but rather need only include a grid or other mechanism for stabilizing the tissue within the biopsy window of the compression paddle. In one embodiment the grid is provided as a detachable component which can be added to existing biopsy compression paddles using latches or other coupling mechanisms. Figure 12B illustrates one embodiment of such a detachable grid. The illustrated grid comprises a generally diamond shaped grid molded of a radioopaque material such as polycarbonate or other plastic. The grid is adapted to removably attach, via snaps, latches, mated grooves, etc., to the compression paddle. The grid provides the ability to stabilize the breast without interfering with the biopsy procedure.

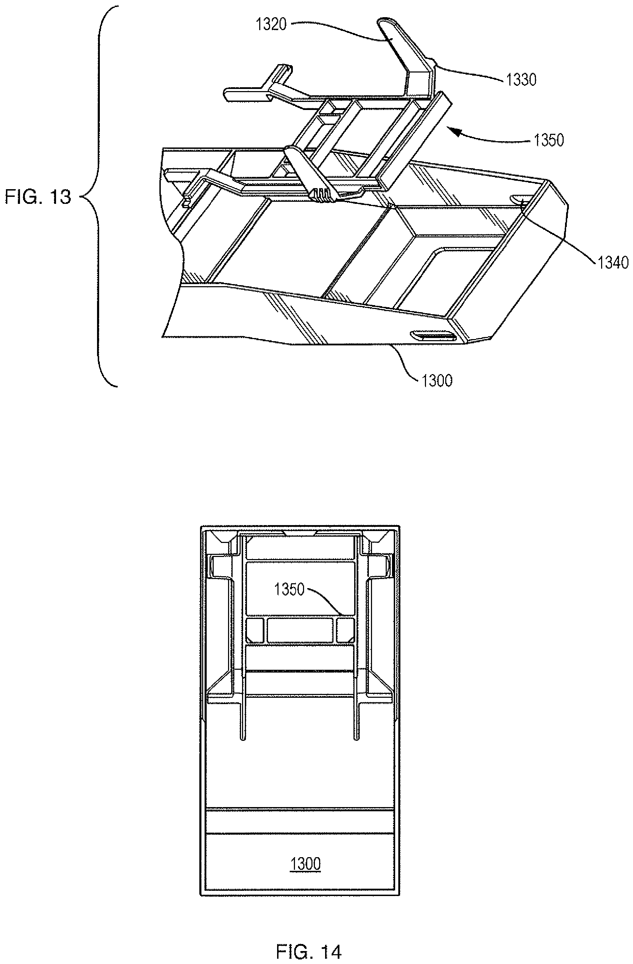

Figures 13 and 14 illustrate another embodiment of a detachable grid 1350 for use with a compression paddle 1300. The detachable grid 1350 comprises lateral slats, similar to those described in Figures 4 and 5, and levers 1320 including tabs 1330 which lock into slots 1340 of the compression paddle 1300. Figure 14 is a top down view showing the detachable grid mounted on the paddle. One advantage of the detachable grids of Figures 12-14 are that they may be removed during the biopsy procedure, should the need arise, without removing the patient from compression. While it is not necessary that the detachable grids have features sized to accept a needle guide, it can be readily appreciated that such spacing would increase the utility of the detachable grid.

Figure 15 illustrates several components that may be included with a compression paddle in an x-ray needle biopsy kit of the present invention. The components include a localizing obturator 1510, an introducer stylet 1512, an introducer sheath 1514 and a needle guide 1516. Although not shown in Figure 15, the components may also include a marker stylet for depositing a radioopaque marker into the biopsy location. In one embodiment the components of Figure 15 are similar to those provided in the ATEC MRI biopsy kit. An x-ray biopsy kit could therefore include the components from the existing MRI biopsy kit together with a biopsy compression paddle. The MRI components may be modified as described above to include fiducials.

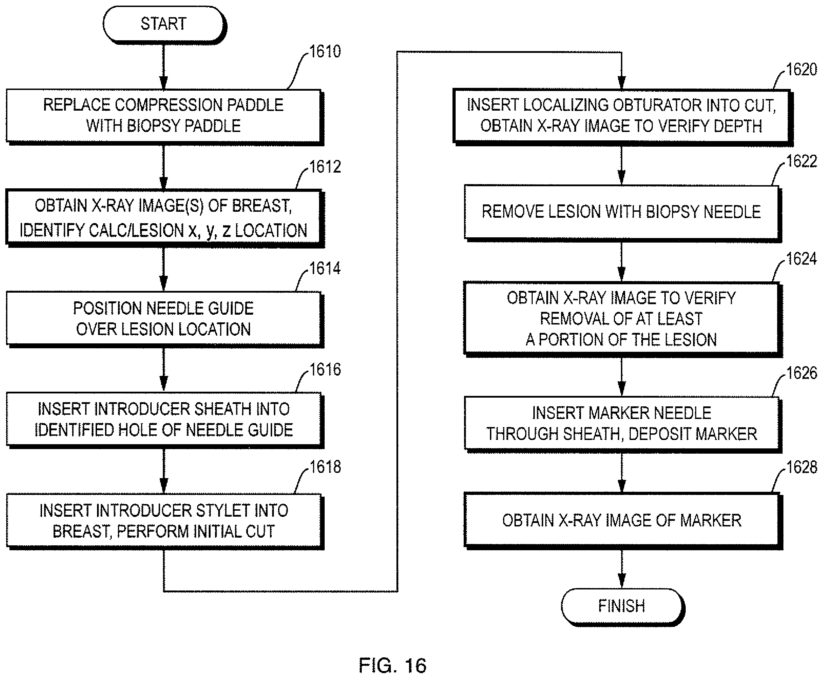

Figure 16 is a flow diagram illustrating exemplary steps that may be performed in a process of the present invention. At step 1610 a compression paddle of an x-ray imaging device is replaced with a biopsy compression paddle (or a detachable grid is added) such as described in conjunction with Figures 4-14. At step 1612 the breast is x-rayed to identify the location of the lesion. This x-ray may be a low dose scout x-ray, a 2D mammogram, a tomosynthesis image, etc. Software on the imaging workstation translates the lesion location into x-y coordinates of the paddle.

At step 1614 the needle guide is positioned in the paddle over (or next to, in the case of a side biopsy) the location of the lesion. At step 1616 the introducer sheath is inserted into the identified hole of the needle guide, and the rubber stopper of the introducer sheath moved to the appropriate, desired depth of cutting. At step 1618 the initial cut into the biopsy location is performed using the introducer stylet, wherein the depth of cut of the introducer stylet is limited based on the position of the rubber stopper of the introducer sheath. The introducer stylet is subsequently removed from the incision.

At step 1620, the localizing obturaror is inserted into incision caused by the introducer stylet and an x-ray is taken to view the tip of the obturator. In one embodiment, the tip of the introducer stylet is imbedded with a fiducial to facilitate identification of the z-axis location of the obturator. The desired location of the tip of the obturator is associated with the notch of a biopsy needle. If the obturator image indicates that the depth is incorrect, steps 1616-1620 are repeated until the desired depth is obtained. At step 1622 a biopsy needle may then be used to remove core samples from the breast.

An example of a biopsy needle that may be used is the ATEC vacuum assisted core biopsy needle which is capable of taking multiple core samples, although the present invention is not limited to use with any particular biopsy device. At step 1624 the breast is again x-rayed to verify removal of at least a portion of the suspicious tissue. At steps 1626 and 1628 a marker may be inserted where the tissue was excised and an x-ray image taken to ensure that the marker is visible.

Thereafter the breast is decompressed and the patient is attended to using standard procedures.

Accordingly an improved system and method for performing a needle biopsy has been presented which allows core samples to be obtained with accuracy using low cost components in conjunction with an x-ray imaging system capable of providing depth information. Research has shown that with such a system, up to twelve core samples can be obtained during a time frame ranging from 15 to twenty minutes; a drastic improvement in efficiency and cost as compared to what is currently available in the x-ray and MRI breast biopsy art.

Having described exemplary embodiments, it can be appreciated that the examples described above are only illustrative and that other examples also are encompassed within the scope of the appended claims.

## Claims

1. Assembly of a compression paddle (100) and a detachable needle guide (500, 910, 1020, 1516), wherein the detachable needle guide has one or more guide holes extending therethrough, the guide holes being arranged for insertion of one or more biopsy devices, the compression paddle comprising:
- a biopsy window,
- a support structure (120) positioned across the biopsy window that has a grid (150, 620, 900, 1350) comprising holes sized to accept the detachable needle guide, and
- a connection mechanism (630) for attaching the compression paddle (100) to a compression mechanism of an upright x-ray imaging system (8) so that a plane of the compression paddle is generally horizontal,
wherein the grid is further adapted to adjustably position the needle guide within the biopsy window, **characterized in that**
the compression paddle and needle guide include mechanical means (914, 1030) for enabling the needle guide to be latched onto or introduced in the compression paddle such that its through holes extend parallel to and below the plane of the compression paddle, allowing for biopsying a breast from the side when the breast is compressed by the compression paddle and attached to the upright x-ray imaging system.

2. The assembly of claim 1 wherein the support structure (120) is detachable from the compression paddle (100).

3. The assembly as claimed in claim 1 or claim 2 wherein the support structure (120) further comprises a plurality of spaced lateral slats.

4. The assembly as claimed in any one of claims 1 to 3 wherein the support structure (120) is radiolucent.

5. The assembly as claimed in any one of claims 1 to 4 further comprising at least one fiducial (800) mounted on the support structure (120).

6. The assembly as claimed in claim 1, wherein the grid is a detachable grid (1210), the detachable grid (1210) comprising: at least one slat spanning at least a portion of the detachable grid; and at least one connector for detachably connecting the detachable grid (1210) across the biopsy window of the breast biopsy compression paddle (100) to enable the at least one slat to secure a portion of a breast extending through the window during a biopsy procedure.

7. The assembly as claimed in claim 6, wherein the at least one slat is radiopaque, and/or wherein the slat extends across a length of the biopsy window, and/or wherein the slat extends at an angle across the biopsy window.

8. The assembly as claimed in any one of claims 1-7, wherein the compression paddle is disposable and releasable coupled to the compression system.

9. The assembly according to claim 1 wherein the needle guide is arranged to be positioned such that the holes of the needle guide (910a) extend below and parallel to the plane of the compression paddle to enable lateral biopsy of the breast.

10. The assembly according to claim 9 wherein the needle guide (1020) is arranged to be latched or coupled to the edge of the compression paddle, such that one or multiple rows of through holes of the needle guide are made available for biopsying the breast from the side.

11. The assembly of a compression paddle according to any of the previous claims, wherein one or more fiducials are embedded in the needle guide.

12. The assembly according to any of the previous claims 1-11, wherein the needle guide comprises at least a row of 2 or more guide holes.

13. A biopsy kit comprising the assembly of a compression paddle (100) of any one of claims 1 to 12 and at least one of an introducer sheath (1514), a introducer stylet (1512), and an obturator (1510).

## Patentansprüche

1. Anordnung eines Kompressionspaddles (100) und einer abnehmbaren Nadelführung (500, 910, 1020, 1516) wobei die abnehmbare Nadelführung ein oder mehrere Führungslöcher aufweist, die sich durch dieselbe erstrecken, wobei die Führungslöcher zum Einführen in eine oder mehrere Biopsievorrichtungen angeordnet sind, das Kompressionspaddle umfassend:
- ein Biopsiefenster,
- eine Trägerstruktur (120)
positioniert über das Biopsiefenster, das ein Raster aufweist (150, 620, 900, 1350), das Löcher umfasst, die so dimensioniert sind, dass sie die ablösbare Nadelführung aufnehmen können, und
- einen Verbindungsmechanismus (630) zum Befestigen des Kompressionspaddles (100) an einem Kompressionsmechanismus eines aufrecht stehenden Röntgenbildgebungssystems (8), sodass eine Ebene des Kompressionspaddles im Allgemeinen horizontal ist,
wobei das Raster ferner zum einstellbaren Positionieren der Nadelführung innerhalb des Biopsiefensters ausgebildet ist, **dadurch gekennzeichnet, dass** das Kompressionspaddle und die Nadelführung mechanische Mittel (914, 1030) umfassen, um zu ermöglichen, dass die Nadelführung so in das Kompressionspaddle eingeklinkt oder eingeführt wird, dass sich ihre Durchgangslöcher parallel zu und unterhalb der Ebene des Kompressionspaddles erstrecken um das Biopsieren einer Brust von der Seite zu ermöglichen, wenn die Brust vom Kompressionspaddle zusammengedrückt und am aufrechtstehenden Röntgenbildgebungssystem befestigt ist.

2. Anordnung nach Anspruch 1, wobei die Trägerstruktur (120) vom Kompressionspaddle (100) abnehmbar ist.

3. Anordnung nach Anspruch 1 oder Anspruch 2, wobei die Trägerstruktur (120) ferner mehrere beabstandete laterale Leisten umfasst.

4. Anordnung nach einem der Ansprüche 1 bis 3, wobei die Trägerstruktur (120) strahlendurchlässig ist.

5. Anordnung nach einem der Ansprüche 1 bis 4, die ferner mindestens eine Bezugsmarkierung (800) umfasst, die an der Trägerstruktur (120) angebracht ist.

6. Anordnung nach Anspruch 11, wobei das Raster ein abnehmbares Raster (1210) ist, das abnehmbare Raster (1210) umfassend: mindestens eine Leiste, die zumindest einen Abschnitt des abnehmbaren Rasters überspannt; und mindestens ein Verbindungsglied zum abnehmbaren Verbinden des abnehmbaren Rasters (1210) über das Biopsiefenster des Brustbiopsie-Kompressionspaddles (100) hinweg, um zu ermöglichen, dass die mindestens eine Leiste einen Abschnitt einer Brust fixiert, der sich während eines Biopsievorgangs durch das Fenster erstreckt.

7. Anordnung nach Anspruch 6, wobei die mindestens eine Leiste röntgenologisch sichtbar ist und/oder wobei sich die Leiste über eine Länge des Biopsiefensters erstreckt, und/oder wobei sich die Leiste in einem Winkel über das Biopsiefenster erstreckt.

8. Anordnung nach einem der Ansprüche 1-7, wobei das Kompressionspaddle entsorgbar und ablösbar mit dem Kompressionssystem verbunden ist.

9. Anordnung nach Anspruch 1, wobei die Nadelführung so angeordnet ist, dass sie so positioniert ist, dass sich die Löcher der Nadelführung (910a) unter und parallel zur Ebene des Kompressionspaddles erstrecken, um laterale Biopsie der Brust zu ermöglichen.

10. Anordnung nach Anspruch 9, wobei die Nadelführung (1020) so angeordnet ist, dass sie in die Kante des Kompressionspaddles eingeklinkt oder mit dieser verbunden werden kann, sodass eine oder mehrere Durchgangslöcher der Nadelführung zum Biopsieren der Brust von der Seite verfügbar gemacht werden.

11. Anordnung eines Kompressionspaddles nach einem der vorhergehenden Ansprüche, wobei eine oder mehrere Bezugsmarkierungen in die Nadelführung eingebettet sind.

12. Anordnung nach einem der Ansprüche 1-11, wobei die Nadelführung mindestens eine Reihe mit 2 oder mehr Führungslöchern umfasst.

13. Biopsie-Kit, das die Anordnung eines Kompressionspaddles (100) nach einem der Ansprüche 1 bis 12 und mindestens eines aus einer Einführschleuse (1514), einem Einführstiletts (1512) und einem Obturator umfasst (1510).

## Revendications

1. Ensemble d'une plaque de compression (100) et d'un guide d'aiguille détachable (500, 910, 1020, 1516), le guide d'aiguille détachable ayant un ou plusieurs trous de guide s'étendant à travers celui-ci, les trous de guide étant agencés pour l'insertion d'un ou plusieurs dispositifs de biopsie, la plaque de compression comprenant :
- une fenêtre de biopsie,
- une structure de support (120) positionnée en travers de la fenêtre de biopsie, qui a une grille (150, 620, 900, 1350) comprenant des trous dimensionnés pour recevoir le guide d'aiguille détachable, et
- un mécanisme de liaison (630) pour attacher la plaque de compression (100) à un mécanisme de compression d'un système d'imagerie verticale à rayons X (8) de telle sorte qu'un plan de la plaque de compression est généralement horizontal,
la grille étant en outre adaptée pour positionner le guide d'aiguille de manière ajustable à l'intérieur de la fenêtre de biopsie, **caractérisé par le fait que**
la plaque de compression et le guide d'aiguille comprennent des moyens mécaniques (914, 1030) pour permettre au guide d'aiguille d'être verrouillé sur la plaque de compression ou introduit dans celle-ci, de telle sorte que ses trous traversant s'étendent parallèlement au plan de la plaque de compression et au-dessous de celui-ci, et permettant la biopsie d'un sein à partir du côté lorsque le sein est comprimé par la plaque de compression et attaché au système d'imagerie verticale à rayons X.

2. Ensemble selon la revendication 1, dans lequel la structure de support (120) est détachable de la plaque de compression (100).

3. Ensemble selon la revendication 1 ou la revendication 2, dans lequel la structure de support (120) comprend en outre une pluralité de lamelles latérales espacées.

4. Ensemble selon l'une quelconque des revendications 1 à 3, dans lequel la structure de support (120) est transparente aux rayons X.

5. Ensemble selon l'une quelconque des revendications 1 à 4, comprenant en outre au moins un repère (800) monté sur la structure de support (120).

6. Ensemble selon la revendication 1, dans lequel la grille est une grille détachable (1210), la grille détachable (1210) comprenant : au moins une lamelle recouvrant au moins une partie de la grille détachable ; et au moins un raccord pour relier de manière détachable la grille détachable (1210) en travers de la fenêtre de biopsie de la plaque de compression de biopsie de sein (100) pour permettre à l'au moins une lamelle d'immobiliser une partie d'un sein s'étendant à travers la fenêtre pendant une procédure de biopsie.

7. Ensemble selon la revendication 6, dans lequel l'au moins une lamelle est radio-opaque et/ou dans lequel la lamelle s'étend sur une longueur de la fenêtre de biopsie et/ou dans lequel la lamelle s'étend selon un angle en travers de la fenêtre de biopsie.

8. Ensemble selon l'une quelconque des revendications 1 à 7, dans lequel la plaque de compression est couplée de manière remplaçable et libérable au système de compression.

9. Ensemble selon la revendication 1, dans lequel le guide d'aiguille est agencé pour être positionné de telle sorte que les trous du guide d'aiguille (910a) s'étendent au-dessous du plan de la plaque de compression et parallèlement à celui-ci pour permettre une biopsie latérale du sein.

10. Ensemble selon la revendication 9, dans lequel le guide d'aiguille (1020) est agencé pour être verrouillé ou couplé au bord de la plaque de compression, de telle sorte qu'une rangée ou de multiples rangées de trous traversants du guide d'aiguille sont rendues disponibles pour la biopsie du sein depuis le côté.

11. Ensemble d'une plaque de compression selon l'une quelconque des revendications précédentes, dans lequel un ou plusieurs repères sont intégrés dans le guide d'aiguille.

12. Ensemble selon l'une quelconque des revendications 1 à 11, dans lequel le guide d'aiguille comprend au moins une rangée d'au moins 2 trous de guide.

13. Kit de biopsie comprenant l'ensemble d'une plaque de compression (100) selon l'une quelconque des revendications 1 à 12 et au moins un parmi une gaine d'introducteur (1514), un stylet d'introducteur (1512) et un obturateur (1510).
